(19) Europäisches Patentamt
European Patent Office
Office européen des brevets

(11) **EP 0 970 951 A1**

## (12) EUROPEAN PATENT APPLICATION

(43) Date of publication:
**12.01.2000 Bulletin 2000/02**

(21) Application number: **98202315.2**

(22) Date of filing: **09.07.1998**

(51) Int. Cl.7: **C07D 301/12**, C07D 301/02, C07C 29/04, C07C 29/48, B01J 31/18

(84) Designated Contracting States:
**AT BE CH CY DE DK ES FI FR GB GR IE IT LI LU MC NL PT SE**
Designated Extension States:
**AL LT LV MK RO SI**

(71) Applicant:
**K.U. Leuven Research & Development
3000 Leuven (BE)**

(72) Inventors:
• **de Vos, Dirk Emmanuel Jan Erik
2800 Mechelen (BE)**

• **de Wildeman, Stefaan M.A.
8750 Zwevezele (BE)**
• **Jacobs, Pierre Auguste
1755 Gooik (BE)**

(74) Representative:
**Hoorweg, Petrus Nicolaas
Arnold & Siedsma,
Advocaten en Octrooigemachtigden,
Sweelinckplein 1
2517 GK Den Haag (NL)**

(54) **A manganese oxidation catalyst, a method for its preparation and use thereof for catalytic epoxidation, catalytic cis-dihydroxylation and combined expoxidation-cis-dihydroxylation of olefins**

(57) The invention relates to a novel manganese oxidation catalyst of the general formula:
$(LMnX_n)S$ wherein:

- Mn is manganese;
- L is a triazacycloalkane, the carbon skeleton thereof comprising at least 6 C atoms; substituted on two of the three N atoms by a non coordinating substituent, and comprising a covalent link to a support S;
- X is a charge compensating anion selected from the group comprising: $Cl^-$, $Br^-$, $F^-$, $I^-$, $CN^-$, $NCS^-$, $N_3^-$, $ClO_4^-$, $NO_3^-$, $RCOO^-$, $RO^-$, $OH^-$, $O_2^{2-}$, $O^{2-}$, $OOH^-$, $SO_4^{2-}$, $HSO_4^-$, $PO_4^{3-}$, $HPO_4^{2-}$, $H_2PO_4^-$, $RSO_3^-$, $RSO_4^-$, $B_4O_7^{2-}$, oxalate, fumarate, maleate, $PF_6^-$, $BF_4^-$;
- n has any value between 0 and 4;
- S is a solid support material, that is linked to L via a covalent tether, the support material being selected from the following groups: oxides, mixed oxides, zeolites, clays, soluble or insoluble organic polymers, or mixed organic-inorganic solids and to a method for its preparation and further to a method for epoxidation and/or dishydroxylation of olefins in the presence of such a catalyst.

**Description**

The invention relates to a novel mangenese oxidation catalyst and to a method for its preparation and further to a method for epoxidation and/or dihydroxylation of olefins in the presence of such a catalyst.

Certain complexes of Mn have been known for years to be active catalysts for epoxidation of olefins and hydroxylation of saturated alkane C-H bonds. Wellknown examples include the tetramethyl and tetraarylporphyrin Mn complexes. Activity in epoxidation of olefins has been known for some time for Mn complexes of the cyclic ligand 1,4,7-trimethyl-1,4,7-triazacyclononane.

US 5,516,738 and US 5,329,024 describe the catalytic epoxidation of styrene and 4-vinylbenzoic acid with a 20- to 500-fold excess of aqueous hydrogen peroxide. The catalyst is a soluble Mn complex with 1,4,7-trimethyl-1,4,7-triazacyclononane or related triazacycles as the ligand. Additional catalyst compositions are obtained via adsorption of the manganese complex onto a solvent insoluble support.

US 5,756,727 describes the synthesis of chiral catalysts consisting of Mn and chiral triazacyclonane-type ligands.

Chem. Commun., 1997, page 355 describes a heterogenisation method of an active manganese triazacyclononane epoxidation catalyst via surface glycidylation. A complex of Mn and 1,4-bis(2'-hydroxyalkyl)-1,4,7-triazacyclononane ligand on a mesoporous MCM-41 support material is disclosed.

A problem with these known catalysts is that the preparation of 1,2-diols is a difficult reaction, particularly when a cis addition of the two hydroxyl groups to the double bond is desired. There are various methods for trans dihydroxylation, those with W and $H_2O_2$ being among the most convenient. Methods for cis dihydroxylation from the prior art are based on the use of stoichiometric oxidants such as permanganate salts, $RuO_4$, $OsO_4$, or on catalytic procedures.

Few catalyst-oxidant combinations are known to be effective, all involving Ru or Os. For instance, Os is an effective catalyst in combination with hydrogen peroxide, metal chlorates, tertiary butyl hydroperoxide, N-methylmorpholine-N-oxide, periodate, oxygen or sodium hypochlorite. Ru catalysts are effective with inter alia periodate.

Even if these methods are catalytic, the use of the expensive and toxic metals Ru and Os is a serious drawback. Moreover, overoxidation of the diol to diones or even oxidative cleavage of the diol can occur as undesired side reactions. Particularly Ru-periodate reactions suffer from this disadvantage.

Therefore it is an object of the present invention to provide a novel catalyst and methods for epoxidation and dihydroxylation, in particular vicinal cis dihydroxylation of olefins with a convenient oxidant.

A further object of the present invention is to provide a novel catalyst and a method for epoxidation and dihydroxylation of olefins which achieves improved yield, conversion and cis selectivity over known catalyst systems.

These objects are achieved by a Mn containing catalyst of the general formula:
$(LMnX_n)S$ wherein:

- Mn is manganese;
- L is a triazacycloalkane, the carbon skeleton thereof comprising at least 6 C atoms; substituted on two of the three N atoms by a non coordinating substituent, and comprising a covalent link to a support S;
- X is a charge compensating anion chosen from the group, comprising: $Cl^-$, $Br^-$, $F^-$, $I^-$, $CN^-$, $NCS^-$, $N_3^-$, $ClO_4^-$, $NO_3^-$, $RCOO^-$, $RO^-$, $OH^-$, $O_2^{2-}$, $O^{2-}$, $OOH^-$, $SO_4^{2-}$, $HSO_4^-$, $PO_4^{3-}$, $HPO_4^{2-}$, $H_2PO_4^-$, $RSO_3^-$, $RSO_4^-$, $B_4O_7^{2-}$, oxalate, fumarate, maleate, $PF_6^-$, $BF_4^-$;
- n has any value between 0 and 4;
- S is a solid support material, that is linked to L via a covalent tether, the support material being selected from the following groups: oxides, mixed oxides, zeolites, clays, soluble or insoluble organic polymers, or mixed organic-inorganic solids.

Preferred examples of non coordinating substituents on the N atoms at positions 1 and 4 are alkyl, aralkyl, aryl, haloalkyl, ethers and esters.

An adversative example of a coordinating substituent, which substituent causes a distortion of the $Mn^{2+}$ coordination is 2-hydroxyalkyl, as disclosed in Chem. Commun., 1997, page 356.

Most preferred examples of non coordinating substituents according to the invention are alkyl, in particular methyl and ethyl.

SUMMARY OF THE INVENTION

The present invention relates in particular to a novel heterogeneous catalyst and its preparation by covalent attachment of a 1,4 substituted 1,4,7-triazacycloalkane ligand (e.g., 1,4-dimethyl-1,4,7-triazacyclononane) to a solid support material. More specifically, a 1,4-dialkyl-1,4,7-triazacyclononane is attached to the solid support via a covalently linking spacer group. The isolated support-bound ligand is metallated in a solution of a Mn salt.

The catalyst is suitable for transformation of olefins into their epoxides, by contacting the olefin with aqueous peroxide and the catalyst in a solvent. Moreover, the catalyst is very useful in the vicinal cis dihydroxylation of olefins with a suitable oxidant, such as $H_2O_2$, without epoxides being formed as intermediate products. Finally, in the presence of a catalyst of the invention, epoxides and cis-diols can be produced simultaneously from olefin reactants.

DESCRIPTION OF THE PREFERRED EMBODIMENTS

Preparation of dmtacn (1,4-dimethyl-1,4,7-triazacyclononane). Dmtacn is relatively easily prepared based on known literature procedures. Starting materials are ethylene glycol and diethylenetriamine. Both products are converted into the tosyl derivatives by standard reaction with tosylchloride. The tritosylate of diethylenetriamine is deprotonated with NaH in dimethylformamide, and reacted with the ditosylate of ethyleneglycol to yield, after purification, pure tritosylated 1,4,7-triazacyclononane (tacn). Two of the three tosyl groups are removed by selective detosylation in HBr/CH$_3$COOH in the presence of phenol. After work up of the monotosylated tacn as a free amine, the two secondary nitrogen groups are methylated with HCHO/HCOOH. Finally the remaining tosyl group is removed in hot sulfuric acid, and after extraction from alkaline solution, dmtacn is obtained as a yellowish oil. The $^1$H NMR spectrum is the characteristic proof for the successful synthesis of dmtacn.

1,4-(di)substituted 1,4,7-triazacycloalkanes with other N-substituents are obtained via similar procedures; e.g. alkylation with 2-bromopropane leads to a doubly isopropylated triazacyclononane.

The next step in the catalyst preparation is the attachment of the dmtacn to a support. This attachment must be of the covalent type. Supports may be organic polymers, semi-organic materials or inorganic polymers. Possible compositions are, amongst others, functionalised polystyrenes, polyacrylonitrile, polyimides, polyamides, polyvinylalcohol. Among the inorganic materials, both crystalline substances (for example clays, zeolites, crystalline oxides) and non-crystalline substances may be used as support material. Preferred support materials are siliceous amorphous materials with the approximate formula SiO$_2$, the number of surface silanol groups being important for the surface coupling reaction. Alternatively mesoporous, hexagonally ordered siliceous materials are suitable supports; these materials are described in patent and open literature. The type of silica (precipitated or pyrogenic) is not critical to the reaction; however, it is advisable that the supports have a considerable specific surface area (for example >100 m$^2$ per g).

Functionalisation of a support with an organic group is a well-known procedure. In the case of the siliceous supports, a convenient method is to add to a suspension of the support in a solvent (preferably toluene) a reagent of the following formula (A):

$$(R^1)_x(R^2O)_ySi(CH_2)_nR^3$$

with R$^1$, R$^2$ any radical
with x + y = 3 , with y minimal 1
with n > 0
with R$^3$ any organic radical that reacts easily with a secondary amine, e.g. an oxirane, or a haloalkyl, or a sulfonatoalkyl.

Examples of suitable reagents are (3-glycidyloxypropyl)trimethoxysilane, (3-chloropropyl)trimethoxysilane or (3-bromopropyl)triethoxysilane. Typically between 0.5 and 5 mmol of the reagent A is used per gram of support. The support may be dehydrated to various extents before suspending in the reaction medium. The hydration degree will influence the amount of reactive surface groups. Typical reaction conditions are temperatures between 30 and 100 °C, and a reaction time between 4 and 24 h. Typical loadings after a successful surface coupling are between 0.3 and 1.5 mmol of active groups per g of support. The material thus obtained, further denoted as functionalised support, may be isolated from the reaction medium by filtration or centrifugation or other conventional techniques; however this is not strictly necessary.

Alternatively a functionalised support may be purchased, commercially, e.g. 3-chloropropyl silica. There are also fully organic commercial functionalized supports that may be used, e.g. a methacrylamide polymer with oxirane functional groups.

A novel step in the catalyst preparation is the reaction in a suitable solvent of the functionalised support with dmtacn, or with any other 1,4 substituted 1,4,7-triazacycloalkane, for example 1,4-dialkyl, 1,4-diaryl or 1,4-diaralkyl substituted 1,4,7-triazacyclononane. Typically the reaction occurs more readily when the functionalised support contains oxirane groups, as is the case when starting from (3-glycidyloxypropyl)trimethoxysilane. In that case, toluene may be used as the reaction solvent, at temperatures between 30 and 100 °C. When the functionalised support contains other groups, more harsh conditions are required, and the addition of a base (e.g. triethylamine) may be required to bring the reaction to a sufficient degree of completion. The material is then isolated by filtration or centrifugation, and dried in ambient conditions or under vacuum. The material is now denoted as heterogenized ligand. In dry conditions, the material can be stored for months without appreciable effects on its catalytic effectiveness.

Alternatively, the order of the surface reaction may be reversed to obtain a heterogenized ligand. More specifically, dmtacn is first reacted in a suitable solvent, e.g. toluene, with an organosilane reagent of the formula (A). A complex organosilane is then obtained. This organosilane does not need to be isolated from the reaction solution; addition of a support, e.g. a siliceous material, yields, after 4-24 h at 30-100 °C a fully equivalent heterogenized ligand with covalent bonds between ligand and support.

The final step in the catalyst preparation according to the invention is the coordination of Mn to the heterogenized ligand. Therefore the heterogenized ligand (e.g. 100 mg) is suspended in a solution containing for example between 50 and 500 micromoles of a Mn salt. The solvent may be water, alcohols, ethers, or a combination thereof. As to the choice of Mn salt, $MnSO_4.H_2O$ gives excellent results, but chlorides, acetates, nitrates, ethylhexanoate, or any other salt such as $Mn(PF_6)_2$ may be used. After stirring at room temperature for sufficiently long time, the metallated powder is isolated by filtration or centrifugation, and is now denoted as catalyst. Repeated washing to remove traces of unchelated metal does not affect the activity.

A first application of the Mn oxidation catalyst according to the invention is the production of epoxides from olefins. Styrene is a particularly good substrate; elevated conversions can be obtained with an excellent epoxide selectivity (over 90 %). The difference with the prior inventions of patents US 5,329,024, US 5,516738 and US 5,756727, is that in the latter reports, the ligand is not attached to the support via a covalent bond. The use of a catalyst with a covalent bond between ligand and support affords better yields of epoxides on peroxide basis. For instance, a 65 % yield of styrene epoxide is obtained from styrene in acetonitrile with a twofold excess of $H_2O_2$, when the covalently bound ligand is used. With the same amount of dmtacn as a soluble ligand in identical conditions, the styrene epoxide yield is only 5 %, because of pronounced disproportionation of $H_2O_2$. In US patents 5,329,024 and 5,516,738, a 20- to 500-fold excess of peroxide is necessary to achieve epoxide formation.

A second application of the Mn oxidation catalyst according to the invention is the reaction of olefins to form cis dihydroxylated products. A simple glass vessel is sufficient as apparatus. The olefin is dissolved in a solvent, and a catalyst according to the invention is added. The reaction mixture is stirred, and the peroxide is added to start the reaction. Temperatures between minus 10°C and plus 30°C are preferred.

Reaction times are for example between 20 minutes and 8h. The peroxide may be previously diluted in a solvent in order to minimize heats of mixing. Slow addition of the peroxide (e.g. over 20 minutes) may be useful to further improve yields on peroxide basis. (Repeated) addition of extra peroxide easily drives the olefin conversion to over 99 %. As a peroxide source, aqueous hydrogen peroxide of any possible concentration may be used; in our experience, the commercially available 30-35 % aqueous $H_2O_2$ is economical, reactive and safe. Excellent results are obtained with acetonitrile or water or mixtures thereof as the solvent, but any solvent may be selected from following classes: alcohols, ketones, nitriles, ethers, hydrocarbons, halogenated hydrocarbons, aromatics, amides, sulfoxides, or esters. Miscibility of the solvent with aqueous hydrogen peroxide is assumed to positively influence the outcome of the reaction. The order of addition of reagents may be changed freely, with only minor effects on the reaction outcome.

The reaction stoichiometry is not subjected to specific restrictions. It is however advisable that the molar amounts of olefin substrate and hydrogen peroxide be of the same order of magnitude. A suitable catalyst concentration is 10 weight % with respect to olefin mass in the reaction, the catalyst weight including the Mn, dmtacn, linking group and support material. On a molar basis, a typical substrate to Mn ratio would be between 50 and 1000, or more often between 200 and 400. Further adjustments in reaction conditions and stoichiometry are thought to be obvious for those with skill in organic catalysis.

Preferred olefin substrates for the cis dihydroxylation are unsaturated alkyl, alicyclic and alkylaryl hydrocarbons, doubly unsaturated conjugated or unconjugated hydrocarbons, or molecules with multiple double bonds, or similar molecules containing in addition functional groups such as alcohols, ketones, esters, amides, nitriles. Examples not limiting the scope of the invention are butenes, hexenes, styrene, cycloalkenes, limonene, isoprene, vinylcyclohexene, esters of sorbic acid, oleic acid, methyl linoleate, geraniol, cis-2-hexene-1-ol, neryl acetate, methacrylamide.

The present method is different from those described in the patents US 5,329,024, US 5,516738 and US 5,756727; in these methods, a catalyst is used that is not attached via a covalent bond to the surface; moreover, epoxidation of olefins is the only reaction claimed. In any prior art, diols formed in reactions of olefins with $H_2O_2$ and a Mn-triazacyclononane type catalyst are secondary products of the reaction; more specifically, the diols are formed by hydrolysis of the initially formed epoxide. Consequently, the diols are trans addition products with respect to the starting olefin. The present invention is different as the diols are formed directly from the olefin, without intermediate epoxides, the insertion mode being cis with respect to the original olefin configuration.

The method according to the invention is not based on the reactivity of permanganate species, formation of the latter being impossible for thermodynamic reasons from Mn cations and hydrogen peroxide. Moreover, the familiar permanganate methods are not catalytic, whereas in the present invention, hydrogen peroxide is used as the oxidant with a catalytic amount of Mn (0.1 - 0.01 mole of Mn per mole of cis diol formed).

It must also be stressed that the covalent linking of the ligand to the support is an essential condition in obtaining cis diols. Procedures in which solutions of non-anchored dmtacn and Mn were used as the catalyst, failed to produce any detectable cis diol in our hands.

The yield of cis dial varies with the substrate type. Generally yields are obtained between 10 and 40 %, at olefin conversions between 20 and 100 %. The stereoselectivity is excellent; the cis addition product far outweighs the trans addition product. A typical cis: trans ratio would be 95 : 5. Overoxidation of diols to hydroxyketones, diketones or further oxidation products is limited; typically the diol : dione ratio is 10.

A final application of the catalyst accoding to the invention is the simultaneous production of epoxide and (cis)-dihydroxylation product from the olefin. This reaction is performed in a mixture of the catalyst and the olefin to which the hydrogen peroxide is added. It is to be underlined that in standard reaction conditions (0 °C, 1-4 h), epoxide hydrolysis is negligible. The intrinsic and long term stability of epoxides in the reaction conditions was proven by exposure of the epoxide (e.g. 1,2-epoxycyclohexane) to standard reaction conditions including the peroxide, the conversion being zero or a few percent at most for long periods (24 h and more) at unusually high temperatures (20 °C). Hence contamination of the cis dihydroxylation product with trans dihydroxylation product is avoided. Thus the maximum yield of the cis dihydroxylation product is obtained long before substantial hydrolysis of the epoxide may lead to appreciable formation of the trans dihydroxylation product.

The following examples will more fully illustrate selected embodiments of the invention.

EXAMPLE 1. Preparation of a heterogenized ligand according to the invention

0.3 g commercial chloropropyl silica (Akros, 0.7 meq Cl per g) is dried for 4h at 120 °C (< 1 mbar). The powder is added to a solution of 0.066 g dmtacn in 20 ml degassed and zeolite-dried toluene. 0.021 g triethylamine is added, and the suspension is stirred for 12 h at 90 °C. After solvent washing (methanol, diethyl ether), the powder is collected in a soxhlet thimble and extracted for 24 h with a diethyl ether - dichloromethane solvent mixture. The supported ligand is then collected by filtration, air dried and stored in a desiccator.

EXAMPLE 2. Preparation of a heterogenized ligand according to the invention

1 g of commercial Silica Gel 60 (Fluka, 70-230 mesh) is dried overnight at 120 °C under vacuum (< 1 mbar). The powder is transferred under inert atmosphere into a flask containing 0.25 g (3-glycidyloxypropyl)trimethoxysilane in 20 ml of dry (zeolite 4A) and degassed toluene. The suspension is stirred for 24 h at 90 °C, and after cooling, 0.12 g dmtacn, dissolved in a small volume of toluene, is added. After 20 h reaction at 60 °C in inert atmosphere, the powder is isolated by filtration over a soxhlet thimble, and extracted in a soxhlet apparatus for 24 h with a solvent mixture such as 50 volume % $CH_2Cl_2$ - 50 volume % EtOEt. The isolated catalyst powder is air dried and stored in a desiccator.

EXAMPLE 3. Preparation of a heterogenized ligand according to the invention

0.25 g (3-glycidyloxypropyl)trimethoxysilane and 0.12 g dmtacn are dissolved in 30 ml dry toluene (dried on zeolite A and $N_2$ flushed), and the solution is heated for 20 h at 60 °C under inert atmosphere. 1 g of vacuum-dried Silica Gel 60 (Fluka; dried under < 1 mbar at 120 °C overnight) is added to the solution. The resulting suspension is heated at 90 °C for 24 h in inert conditions. The powder is collected in a soxhlet extraction thimble, and extracted for 24 h with a diethyl ether - dichloromethane solvent mixture. The supported ligand is then collected by filtration, air dried and stored in a desiccator.

EXAMPLE 4. Preparation of a heterogenized ligand according to the invention

A mesoporous, hexagonally ordered siliceous material is prepared based on a literature procedure. Any organic molecules are removed from the structure, first by extraction with hot ethanol, subsequently by slowly heating in air and overnight calcination at 550 °C. 1 g of this material is taken and treated in a fully similar way as the Silica Gel in Example 2. Further specifications are given in Example 2.

EXAMPLE 5. Preparation of a heterogenized ligand according to the invention

1.0 g of Eupergit® C 250 L (commercially available from Fluka) is brought into 20 ml of absolute ethanol, and 30 mg dmtacn (dissolved in 1 ml EtOH) is added. The mixture is stirred for 4 h at 50 °C, and the polymer is recovered by filtration. The polymer is dried under a flow of dry nitrogen and stored at -10 °C in an inert atmosphere.

EXAMPLE 6. Metallation of the heterogenized ligand

A water solution of $MnSO_4.H_2O$ is prepared, containing 0.067 g of salt per 10 ml water. 10 mg of the supported ligand is stirred for 1 h in 1 ml MeOH and 250 microliter of the aqueous Mn solution. After centrifugation, the supernatant is removed and the obtained catalyst is air dried.

EXAMPLE 7. Epoxidation of styrene

A heterogenized ligand is prepared based on a hexagonally ordered mesoporous silica, as described in Example 4. The heterogenized ligand is metallated as described in Example 6, and 10 mg of the resulting catalyst powder is mixed with 0.18 g styrene and 1 ml of acetone. 0.35 g of 35 % aqueous $H_2O_2$ is added to the stirred suspension. After 100 minutes, 33 % of the styrene is converted, with a selectivity of 92 % for styrene epoxide.

EXAMPLE 8. Epoxidation of styrene

10 mg of a heterogenized ligand according to Example 2 is metallated following the procedure of Example 6, and is mixed with 0.045 g styrene, 1 ml acetonitrile and 0.086 g of 35 % H$_2$O$_2$. After 20 minutes at 0°C, styrene is converted for 72 %, with a selectivity of 91 % for the epoxide.

EXAMPLE 9. Cis-dihydroxylation of cis-2-hexene

A heterogenized ligand is prepared according to Example 3 and further metallated according to Example 6 in order to obtain the catalyst. To 10 mg freshly metallated catalyst are added 0.084 g cis-2-hexene and 1 ml of CH$_3$CN. The suspension is stirred at 0 °C, and 2 mmol of 35 % aqueous H$_2$O$_2$ (0.2 g) diluted in 0.4 ml CH$_3$CN, is added through a syringe. Based on GC analysis, the cis-2-hexene is converted for 60 % after 1 h, with a 35 % molar selectivity for 2,3-hexanediol. The cis : trans ratio in the diol fraction (considered with respect to the starting cis configuration of the olefin) is 94 to 6. Overoxidation to diones and related products is only 4 mole %.

EXAMPLE 10. Cis-dihydroxylation of cis-2-hexene

The heterogenized ligand was prepared as in Example 3, and metallated as in Example 6. To 10 mg freshly metallated catalyst are added 0.084 g cis-2-hexene and 1 ml of acetone. The suspension is stirred at 0 °C, and 2 mmol of 35 % aqueous H$_2$O$_2$, diluted in 0.4 ml acetone, is added through a syringe. Based on GC analysis, a 28 % molar selectivity for 2,3-hexanediols is obtained after 1 h, at a cis-2-hexene conversion of 71 %. The cis : trans ratio in the diol fraction (considered with respect to the starting cis configuration of the olefin) is 92 to 8. Overoxidation to diones and related products is 8 mole %.

EXAMPLE 11. Cis-dihydroxylation of trans-2-hexene

The procedure followed was identical to that of Example 9, with trans-2-hexene instead of cis-2-hexene as the starting olefin. After 20 minutes, the olefin conversion was 18 %, with a diol selectivity of 37 %. In the diol fraction, the ratio between cis and trans dihydroxylation products was 94 to 6. Thus the main diol (the cis-dihydroxylated product of trans-2-hexene) is the same as the minoritary diol in Example 9, where the minor product is the trans-dihydroxylation product of cis-2-hexene.

EXAMPLE 12. Cis-dihydroxylation of norbornene

The heterogenized ligand is prepared as in Example 3, and metallated as in Example 6. To 10 mg freshly metallated catalyst are added 0.094 g norbornene and 1 ml of CH$_3$CN. The suspension is stirred at 0 °C, and 2 mmol of 35 % aqueous H$_2$O$_2$, diluted in 0.4 ml CH$_3$CN, is added through a syringe. Based on GC analysis, a 32 % molar selectivity for vicinal diols is obtained after 5 h, at a norbornene conversion of 85 %. All the diols formed have the cis configuration with respect to norbornene; products of trans dihydroxylation are not formed. Overoxidation to diones and related products amounts to only 3.4 mole % of the converted norbornene.

EXAMPLE 13. Combined epoxidation and cis-dihydroxylation of cyclohexene

A catalyst is prepared as in Example 3. To 10 mg freshly metallated catalyst are added 0.082 g cyclohexene and 1 ml of CH$_3$CN. The suspension is stirred at 0 °C, and 2 mmol of 35 % aqueous H$_2$O$_2$, diluted in 0.4 ml CH$_3$CN, is added through a syringe. Based on GC analysis, the conversion of the olefin amounts to 43 % after 20 minutes. The epoxide selectivity on a molar basis is 78 %. Selectivity for 1,2-cyclohexanediols is 12 %. The cis : trans ratio in the diol fraction is 99 to 1. Overoxidation to diones and related products is not detected. After 2 h, olefin conversion is 96 %, with an epoxide selectivity of 62 % and a selectivity for cis-1,2-cyclohexanediol of 17 %.

EXAMPLE 14. Cis-dihydroxylation of cis-2-hexene with a catalyst containing Mn and covalently linked 1,4-dibenzyl substituted 1,4,7-triazacyclononane. 1,4-Dibenzyl-1,4,7-triazacyclononane was synthesized starting from 1-tosyl-1,4,7-triazacyclononane. The latter is also an intermediate in dmtacn synthesis. 1-Tosyl-1,4,7-triazacyclononane was benzylated at positions 1 and 4 using 2 equivalents of benzyl bromide, and an excess of base to neutralize the HBr formed. The tosyl group was removed in hot sulfuric acid, and the resulting 1,4-dibenzyl-1,4,7-triazacyclononane (dbtacn) was worked up. A supported ligand was prepared starting from dbtacn following a procedure analogous to the one described in Example 3, and metallated according to Example 6. Catalytic cis-dihydroxylation was performed as described in Example 9. A final conversion of 32% was obtained, with a cis-diol selectivity of 33%. Trans-diol selectivity was less than 4%.

EXAMPLE 15. Attempted cis-dihydroxylation of cis-2-hexene with homogeneous Mn-dmtacn complexes

(negative example, vs. Example 9. This example demonstrates that a homogeneous dmtacn ligand is not suitable for being used in a cis-dihydroxylation).

Dmtacn (0.5 mg) and MnSO$_4$ (0.33 mg) were mixed in a solvent consisting of 0.1 ml water and 1 ml of acetonitrile, in order to obtain a homogeneous catalyst. To this stirred solution (0°C) were added 0.084 g of cis-2-hexene and 0.2 g of a 35 weight % H$_2$O$_2$ solution. Oxygen evolution was rapid. After 60 minutes, cis-2-hexene conversion was less than 5 %, and the product of cis-dihydroxylation was not detected in the reaction mixture. After longer periods, the reaction did not show any progress, and no residual peroxide could be detected in the reaction mixture.

EXAMPLE 16. Attempted cis-dihydroxylation of cis-2-hexene with Mn-dmtacn adsorbed onto a support without covalent linking.

(negative example, vs. Example 9. This example demonstrates that heterogenization of the Mn-dmtacn complex by simple physisorption onto an inorganic support does not lead to cis-dihydroxylation).
Dmtacn (0.5 mg) and MnSO₄ (0.33 mg) were mixed in a solvent consisting of 0.1 ml water and 1 ml of acetonitrile, in order to obtain a Mn-dmtacn complex. To the solution was added 100 mg of a commercial Silica-Alumina material, and the suspension was stirred for 1 h. After cooling to 0°C, 0.084 g of cis-2-hexene and 0.2 g of a 35 weight% H₂O solution were added. Rapid oxygen evolution was observed; moreover the supernatant was of a clearly brown color, indicating that the association of the complex with the surface was not ensured. After 120 minutes, the yield of cis-2-hexene epoxide was less than 3%, and no cis-dihydroxylated product was detected at all.

EXAMPLE 17. Attempted cis-dihydroxylation of cis-2-hexene with a catalyst with 2-hydroxypropyl coordinating substituent.

(This example demonstrates that coordinating substituents on the N atoms are not suitable to produce cis-diols. It is particularly striking that even when using more catalyst, substrate conversions are very low).
1 g of a silica surface was functionalized with (3-glycidyl)oxypropyltrimethoxysilane as previously described, and subsequently reacted with 0.10 g commercial 1,4,7-triazacyclononane (toluene, 90°C, 24h). The suspension was cooled to room temperature and 0.3 g propylene oxide was added. After another 10 h of stirring, the powder was isolated and purified as usual, and finally metallated as in Example 6. The catalyst was used in a reaction with cis-2-hexene as described for Example 9, except that a double amount of catalyst was used (20 mg). The reaction was followed until the yields did not progress any longer. Only the epoxide (5 % yield) and epoxide-derived products, e.g. the trans-dihydroxylation product (less than 1 % yield) were detected. No cis diol was detected.

## Claims

1. A Mn containing catalyst of the general formula:
   (LMnX$_n$)S wherein:

   - Mn is manganese ;
   - L is a triazacycloalkane, the carbon skeleton thereof comprising at least 6 C atoms; substituted on two of the three N atoms by a non coordinating substituent, and comprising a covalent link to a support S;
   - X is a charge compensating anion selected from the group comprising: Cl⁻, Br⁻, F⁻, I⁻, CN⁻, NCS⁻, N₃⁻, ClO₄⁻, NO₃⁻, RCOO⁻, RO⁻, OH⁻, O₂²⁻, O²⁻, OOH⁻, SO₄²⁻, HSO₄²⁻, PO₄³⁻, HPO₄²⁻, H₂PO₄⁻, RSO₃⁻, RSO₄⁻, B₄O₇²⁻, oxalate, fumarate, maleate, PF₆⁻, BF₄⁻;
   - n has any value between 0 and 4;
   - S is a solid support material, that is linked to L via a covalent tether, the support material being selected from the following groups: oxides, mixed oxides, zeolites, clays, soluble or insoluble organic polymers, or mixed organic-inorganic solids.

2. Catalyst according to claim 1, wherein L is 1,4-substituted 1,4,7-triazacyclononane, substituted on the N atoms at positions 1 and 4 by a non-coordinating substituent selected from the group comprising alkyl, aralkyl, aryl, haloalkyl, ethers and esters and comprising the covalent link to the support at position 7.

3. Catalyst according to claim 2, wherein L is 1,4-dimethyl 1,4,7-triazacyclononane, L is 1,4-diethyl 1,4,7-triazacyclononane or 1,4-dibenzyl 1,4,7-triazacyclononane.

4. Catalyst according to one of the claims 1-3, wherein L = dmtacn (1,4-dimethyl-1,4,7-triazacyclononane), X=SO₄²⁻, n=1, S is an amorphous SiO₂ support and the covalent group between S and L has the formula (S)-(CH₂)₃-O-CH₂-CH(OH)-CH₂-(L); L being attached to the non-methylated N atom at position 7 of dmtacn.

5. Catalyst according to claims 1-4, wherein the covalently linking group between the support and L is -(CH₂)$_n$-O-CH₂-CH(OH)-CH₂-, -(CH₂)$_n$-CH(OH)-CH₂-, -(CH₂)$_m$-, -(CH₂)$_n$C(=O)-, -(CH₂)$_n$C(=O)O-,; L being attached at the latter end of the covalently linking group (n=0-30; m=1-30).

6. A method for the preparation of a catalyst according to claims 1-5, comprising the synthesis of the ligand 1,4-substituted 1,4,7-triazacyclononane, the functionalisation of a support with a reactive organic group, and the coupling of the ligand to the functionalised support via a covalent bond.

7. A method for epoxidation of an olefin, that comprises contacting a catalyst according to one of the claims 1-5 with an olefin and an oxidizing agent.

8. A method for formation of a vicinal diol from an olefin, comprising contacting the olefin with an oxidizing agent, in the presence of a Mn catalyst according to one of the claims 1-5, wherein the cis dihydroxylation is the preferred reaction.

9. A method according to claim 8, wherein one the two -OH groups to be inserted is replaced with a -OR, -$NH_2$, -NHR, $NR_2$, -Cl, or -F; the insertion of the latter groups being obtained by addition of respectively HOR, $H_3N$, $H_2NR$, $HNR_2$, or a source of chloride or fluoride to the reaction medium.

10. A method according to claim 8, wherein one the two -OH groups to be inserted is replaced with a N containing radical, resulting from the addition of N-bromoacetamide or chloramine-T to the reaction medium.

11. A method for the oxidation of an olefin to a mixture of the epoxide and the cis-dihydroxylation product; the method consists in adding a catalyst according to claims 1-5 to a mixture of the olefin and hydrogen peroxide.

12. Method according to claims 7-11 wherein the reaction is performed in a solvent belonging to one of following solvent groups: water, alcohols, ketones, nitriles, ethers, hydrocarbons, halogenated hydrocarbons, aromatics, amides, sulfoxides, or esters.

13. Method according to claims 7-12, wherein the oxidizing agent is one of the following: hydrogen peroxide, an alkyl hydroperoxide, a peracid, a salt of a peracid, hypochlorite, N-morpholine-oxide, iodosylbenzene, or any precursor that generates in situ one of the forementioned oxidizing agents, e.g. sodium percarbonate, or the adduct of urea and hydrogen peroxide.

## DOCUMENTS CONSIDERED TO BE RELEVANT

| Category | Citation of document with indication, where appropriate, of relevant passages | Relevant to claim | CLASSIFICATION OF THE APPLICATION (Int.Cl.6) |
|---|---|---|---|
| A | GB 2 225 963 A (ATOMIC ENERGY AUTHORITY UK) 20 June 1990 * the whole document * | 1,6,7 | C07D301/12 C07D301/02 C07C29/04 C07C29/48 B01J31/18 |
| D,A | US 5 329 024 A (JURELLER SHARON H ET AL) 12 July 1994 * the whole document * | 1-13 | |
| A | EP 0 751 135 A (HOECHST AG) 2 January 1997 * the whole document * | 1-13 | |

| | | | TECHNICAL FIELDS SEARCHED (Int.Cl.6) |
|---|---|---|---|
| | | | C07D C07C B01J |

The present search report has been drawn up for all claims

| Place of search | Date of completion of the search | Examiner |
|---|---|---|
| THE HAGUE | 8 December 1998 | Rotsaert, L |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**ANNEX TO THE EUROPEAN SEARCH REPORT
ON EUROPEAN PATENT APPLICATION NO.**

EP 98 20 2315

This annex lists the patent family members relating to the patent documents cited in the above-mentioned European search report.
The members are as contained in the European Patent Office EDP file on
The European Patent Office is in no way liable for these particulars which are merely given for the purpose of information.

08-12-1998

| Patent document cited in search report | | Publication date | Patent family member(s) | | Publication date |
|---|---|---|---|---|---|
| GB 2225963 | A | 20-06-1990 | NONE | | |
| US 5329024 | A | 12-07-1994 | CA | 2120268 A,C | 01-10-1994 |
| | | | DE | 69408884 D | 16-04-1998 |
| | | | DE | 69408884 T | 25-06-1998 |
| | | | EP | 0618202 A | 05-10-1994 |
| | | | ES | 2115094 T | 16-06-1998 |
| | | | JP | 2764005 B | 11-06-1998 |
| | | | JP | 6321924 A | 22-11-1994 |
| | | | KR | 9702230 B | 26-02-1997 |
| | | | US | 5516738 A | 14-05-1996 |
| EP 0751135 | A | 02-01-1997 | DE | 19523890 C | 28-11-1996 |
| | | | JP | 9025274 A | 28-01-1997 |
| | | | US | 5792878 A | 11-08-1998 |

EPO FORM P0459

For more details about this annex : see Official Journal of the European Patent Office, No. 12/82